# EUROPEAN PATENT APPLICATION

(11) **EP 3 949 957 A1**
(43) Date of publication of application: **09.02.2022**
(21) Application number: 20775806.1
(22) Date of filing: 16.01.2020
(51) Int. Cl.: A61K 31/047, A61K 31/7004, A61K 31/7008, A61K 31/7012, A61K 31/704, A61K 45/00, A61P 13/02, A61P 13/10, A61P 13/12, A61P 35/00, A61P 43/00, A61K 9/08, A61K 47/54

(54) **PHARMACEUTICAL COMPOSITION INJECTABLE INTO URINARY TRACK ORGAN CAVITY FOR PREVENTION OR TREATMENT OF UROTHELIAL CANCER**

(30) Priority: 26.03.2019 JP 2019058477
(71) Applicant: National University Corporation Kagawa University, Takamatsu-shi, Kagawa 760-8521 (JP)
(72) Inventor: TAOKA, Rikiya, Kita-gun, Kagawa 761-0793 (JP); KAKEHI, Yoshiyuki, Takamatsu-shi, Kagawa 760-8521 (JP); SUGIMOTO, Mikio, Kita-gun, Kagawa 761-0793 (JP); ZHANG, Xia, Kita-gun, Kagawa 761-0793 (JP); MATSUOKA, Yuki, Kita-gun, Kagawa 761-0793 (JP); IZUMORI, Ken, Kita-gun, Kagawa 761-0795 (JP); YOSHIHARA, Akihide, Kita-gun, Kagawa 761-0795 (JP)
(74) Representative: TBK
(86) International application number: PCT/JP2020/001334
(87) International publication number: WO 2020/195037

(57) **Abstract**

Provided is a pharmaceutical composition that is for prevention or treatment of urinary tract organ cancer, causes no adverse side effects, can be continuously used for a long time, and is administered to the urinary tract organ by injection into the urinary tract organ cavity. A solution for injection into the urinary tract organ cavity contains D-allose. The inside of the urinary tract organ cavity is the inside of the upper urinary tract and the inside of the bladder. The solution is for injection therapy into the urinary tract organ cavity. The solution uses enhancement of sugar uptake into cancer cells of the urinary tract organ (for example, RT112, 253J, J82) by D-allose. The solution contains D-allose together with a pharmaceutically acceptable diluent or carrier. The solution is a pharmaceutical composition for prevention or treatment of urinary tract organ cancer.

## Description

### TECHNICAL FIELD

The present invention relates to a pharmaceutical composition containing D-allose in an effective amount, for prevention or treatment of urothelial cancer by injection into the urinary tract organ cavity.

### BACKGROUND ART

Urine produced in the kidneys is discharged to the renal pelvis and is transferred through the ureters to the bladder. When storing a certain amount of urine, the bladder generates uresiesthesia, and the bladder muscle contracts to discharge urine outside the body. Urothelial cancer is the cancer of the renal pelvis, the ureter, and the bladder through which urine passes. About 70% of the cancer is probably non-muscle invasive, and for the treatment, transurethral surgery and injection therapy of Bacillus Calmette-Guerin (BCG) or an anticancer agent into the urinary tract organ cavity are discussed as a standard of care. However, the five-year recurrence rate after the injection therapy using an existing medicinal agent is as high as 31 to 78%, and the five-year recurrence rate after additional injection therapy for the recurrence cases is still 40 to 55%. This indicates that about a half of the cases recur, and the recurrent cases are discussed to resect the urinary tract organ (Non-Patent Document 1). The resection of the urinary tract organ essentially involves reduction in quality of life (QOL) of a patient, including hypofunction of the kidneys due to total nephroureterectomy of an affected side or stoma formation in the abdominal wall for urine due to total cystectomy. There is thus a strong demand for an injection therapy using a novel medicinal agent in order to prevent recurrence and to conserve the urinary tract organ.

The most frequently used medicinal agent in the injection therapy for non-muscle invasive urothelial cancer is BCG. The BCG is derived from Mycobacterium bovis transferred to Albert Calmette and Camille Guerin in 1904. The original strain has an extremely high toxicity, and after subculture more than 230 times in a bile potato medium, an avirulent strain usable as a vaccine was produced. The BCG injection therapy still frequently causes adverse events such as irritation symptom of the bladder and is not completed in many cases, unfortunately. There is thus a demand for a medicinal agent having higher tolerability.

According to the projected cancer statistics in 2018 in Japan, the projected cancer incidence in the kidney, the urinary tract, and the bladder is 52,400 in males and females, and these organs are the 6th most common in the cancers in Japan. In some reports, the onset age of urothelial cancer is 73 years old, and the number of patients tends to increase in the aging society in Japan. In addition, urothelial cancer recurs at a high rate, and the injection therapy for treatment or recurrence prevention is highly frequently performed. In such circumstances, a solution of the present invention promises an improvement in antitumor effect of a medicinal agent to be used or an improvement in tolerability and thus should make a large impact on the society.

In the medical field, the results of the application studies of rare sugars include an invention of an in vivo antioxidant containing D-allose as an active component (Patent Document 1). This antioxidant is used as a composition containing D-allose and is administered to a patient with liver cancer or skin cancer to treat the liver cancer or the skin cancer by in vivo antioxidant activity of the D-allose.

### Related Art Documents

### Patent Documents

Patent Document 1: JP-B No. 5330976
Patent Document 2: JP-B No. 3975274

### Non-Patent Document

Non-Patent Document 1: Clinical practice guideline for bladder cancer, 2015

### SUMMARY

### Technical Problem

As mentioned above, the most frequently used medicinal agent in the injection therapy for non-muscle invasive urothelial cancer is BCG at the present time. It is, however, known that the injection therapy using BCG may fail to treat non-muscle invasive urothelial cancer in some cases, and 40 to 55% of the treated cases still recur. As described above, the injection therapy using BCG has insufficient antitumor effect and frequently causes adverse events in clinical practice to result in a low completion rate, unfortunately.

There is a strong demand for a novel medicinal agent in the injection therapy into the urinary tract organ cavity for non-muscle invasive urothelial cancer.

### Solution to Problem

The inventors of the present invention have conducted intensive studies in order to solve the above problems and have found that D-allose, a rare sugar, has a strong antitumor effect on human urothelial cancer cells. The inventors of the present invention have therefore intended to provide a novel medicinal agent that is a solution containing D-allose and should have a high antitumor effect on urothelial cancer. The present invention provides a pharmaceutical composition containing D-allose for construction of a novel treatment strategy for urothelial cancer, on the basis of a strong antitumor effect of D-allose on urothelial cancer cells, such high safety of D-allose as almost no harmful effect on normal cells, and an organ specific advantage in that a solution containing D-allose is directly injectable through the urethra into the urinary tract organ cavity.

The present invention relates to a solution for injection into a urinary tract organ cavity in the following aspects (1) to (21).
(1) A solution for injection into a urinary tract organ cavity, the solution including D-allose.
(2) The solution according to the aspect (1), in which the D-allose is D-allose and/or a derivative thereof and/or a mixture thereof.
(3) The solution according to the aspect (2), in which the derivative of D-allose is a D-allose derivative selected from a sugar alcohol in which a carbonyl group of D-allose is substituted with an alcohol group, a uronic acid in which an alcohol group of D-allose is oxidized, or an amino sugar in which an alcohol group of D-allose is substituted with an NH₂ group.
(4) The solution according to any one of the aspects (1) to (3), in which an inside of the urinary tract organ cavity is an inside of an upper urinary tract and an inside of a bladder.
(5) The solution according to any one of the aspects (1) to (4), for injection therapy into the urinary tract organ cavity.
(6) The solution according to any one of the aspects (1) to (5), using enhancement of sugar uptake into cancer cells of the urinary tract organ by D-allose.
(7) The solution according to any one of the aspects (1) to (6), in which D-allose is contained in an effective amount.
(8) The solution according to any one of the aspects (5) to (7), to be injected into the urinary tract organ cavity by injection into the urinary tract organ cavity.
(9) The solution according to any one of the aspects (1) to (8), in which D-allose is contained together with a pharmaceutically acceptable diluent or a pharmaceutically acceptable carrier.
(10) The solution according to any one of the aspects (1) to (9), in which one or more antiproliferative agents are further contained.
(11) The solution according to any one of the aspects (1) to (10), in which D-allose is associated with a drug for enhancing uptake into cancer cells of the urinary tract organ.
(12) The solution according to the aspect (11), in which the drug includes an anticancer agent.
(13) The solution according to the aspect (11) or (12), in which D-allose and the drug are associated directly or covalently through a linker.
(14) The solution according to any one of the aspects (11) to (13), in which the drug is a radioisotope, an enzyme, a prodrug-activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.
(15) The solution according to the aspect (10), in which at least one of the antiproliferative agents is an anthracycline.
(16) The solution according to the aspect (15), in which the anthracycline is selected from the group consisting of doxorubicin, epirubicin, daunorubicin, aclarubicin, idarubicin, pirarubicin, annamycin, methoxymorpholinodoxorubicin, cyanomorpholinyldoxorubicin, valrubicin (N-trifluoroacetyladriamycin-14-valerate), and mitoxantrone.
(17) The solution according to the aspect (15), in which the anthracycline is selected from the group consisting of valrubicin, doxorubicin, and epirubicin.
(18) The solution according to the aspect (15), in which the anthracycline is epirubicin.
(19) The solution according to any one of the aspects (6) to (18), in which the urinary tract organ cancer is non-muscle invasive urothelial cancer.
(20) The solution according to any one of the aspects (6) to (19), in which the urinary tract organ cancer is renal pelvic cancer, ureter cancer, bladder cancer, or ureteral cancer.
(21) The solution according to any one of the aspects (1) to (20), being a pharmaceutical composition for prevention or treatment of urinary tract organ cancer.

The present invention also relates to a kit in the following aspect (22).

(22) A kit including the solution as a pharmaceutical composition according to the aspect (21) and a package insert indicating that the pharmaceutical composition is injected into a urinary tract organ cavity of a patient requiring prevention or treatment of urinary tract organ cancer, and accordingly the urinary tract organ cancer of the patient is prevented or treated.

### Advantageous Effects of Invention

Cancer cells have such characteristics as infinite proliferative capacity and metastaticity and thus require a large amount of energy. Cell energy is ATP, and to produce more ATP, cancer cells enhance metabolic pathways contributing energy production, such as glycometabolism, lipid metabolism, and amino acid metabolism. For example, cancer cells commonly enhance sugar uptake, and PET diagnosis is based on this phenomenon in clinical practice. Meanwhile, anticancer agents (such as mitomycin C and adriamycin) conventionally used to treat bladder cancer have selectivity to actively proliferating cancer cells, but the action mechanism is inhibition of protein synthesis or nucleic acid synthesis, and thus the anticancer agents also act on normal cells to some extent to cause adverse side effects.

In contrast, a pharmaceutical product of the present invention that contains D-allose and is used for prevention or treatment of urothelial cancer is less harmful to normal cells. In addition, an injection therapy into the urinary tract organ cavity using a solution containing D-allose of the present invention probably causes a few systemic adverse events because the solution is directly applied to cancer cells and thus the treatment region is limited to the inside of the urinary tract organ cavity. This enables long-term and continuous treatment to improve the treatment effect on urothelial cancer, resulting in an improvement in quality of life (QOL) of a patient.

More specifically, the present invention can provide use of D-allose for construction of a novel prevention or treatment strategy for urothelial cancer. The use achieves high selectivity to urothelial cancer cells and provides almost no harmful effect on normal cells. The present invention can also provide a pharmaceutical composition for prevention or treatment of urinary tract organ cancer. The pharmaceutical composition causes no adverse side effects, can be continuously used for a long time, and is administered to the urinary tract organ by injection into the urinary tract organ cavity. The solution containing D-allose is directly injected through the urethra into the transurethrally urinary tract organ cavity and is applied to urothelial cancer cells, and this can provide a novel treatment method promising to achieve high antitumor effect and safety.

### BRIEF DESCRIPTION OF DRAWINGS

Fig. 1 shows the result of survival assay of a urothelial cancer cell line (RT112).
Fig. 2 shows the result of survival assay of a urothelial cancer cell line (253J).
Fig. 3 shows the result of survival assay of a urothelial cancer cell line (J82).

### DESCRIPTION OF EMBODIMENTS

An "injection therapy into the urinary tract organ cavity" aimed in the present invention means the injection therapy using a solution containing D-allose into the renal pelvis and ureter or the bladder cavity. Depending on localization of urothelial cancer, "ureteropelvic injection therapy" or "intravesical injection therapy" is selected. The injection therapy is performed through a catheter. An "intravesical (ureteropelvic) solution", an "intravesical (ureteropelvic) active substance", an "intravesical (ureteropelvic) therapy", and an "intravesical (ureteropelvic) compound" mean treatment capable of being administered to the bladder (the renal pelvis and ureter). For example, in an embodiment, an intravesical (ureteropelvic) active substance is a pharmaceutical product containing D-allose and a pharmaceutically acceptable diluent or carrier. In another embodiment, an intravesical (ureteropelvic) active substance is a pharmaceutical product containing D-allose, one or more additional antiproliferative agents, and a pharmaceutically acceptable diluent or carrier. In an embodiment, an intravesical (ureteropelvic) therapy is a combination of oral administration and an intravesical active substance. The present invention is not intended to be limited to the combination of oral administration and an intravesical (ureteropelvic) active substance. For example, in an embodiment, the intravesical (ureteropelvic) therapy is an intravesical (ureteropelvic) active substance. In another embodiment, the intravesical (ureteropelvic) therapy is a combination of intravesical active substances, for example, a combination of D-allose and one or more additional antiproliferative agents.

The present invention is a urothelial cancer treatment composition containing D-allose and includes a drug product containing D-allose in an effective amount or a pharmacologically acceptable salt and/or hydrate.

The D-allose used in the present invention is a rare sugar present in an extremely small amount as compared with D-glucose abundant in nature. The basic units of sugars are 34 monosaccharides (monosaccharides having a carbon number of 6: hexoses) including 16 aldoses, 8 ketoses, and 10 sugar alcohols. In contrast to "natural monosaccharides" typified by D-glucose abundant in nature, monosaccharides (aldoses and ketoses) present in trace amounts in nature and derivatives thereof (sugar alcohols) are defined as "rare sugars". Rare sugars capable of being mass-produced at the present time are D-psicose (D-allulose) and D-allose. D-Allose is the D-isomer of allose that is classified into aldoses in hexoses.

The method of producing D-allose includes a synthesis method from D-allulose (D-psicose) with L-rhamnose isomerase and a production method in which a D-allulose-containing solution is reacted with D-xylose isomerase. The method of producing high-purity D-allose includes a fractionation method by crystallization of D-allose (Patent Document 2). The production method is not limited to the above methods, and the D-allose in the present invention may be produced by any method such as an isomerization method by chemical treatment. D-allulose as a material of D-allose is now typically produced by an enzyme (epimerase) treatment method of fructose but may be produced by any method. D-Allulose may be produced by a method using microorganisms producing the enzyme, may be extracted from a natural product or be an intact natural product, or may be an isomerized product by a chemical treatment method. The method of purifying D-allulose by using an enzyme is well-known.

Derivatives of D-allose will be described. A compound converted by chemical reaction of the molecular structure of a starting compound is called a derivative of the starting compound. The derivatives of hexoses including D-allose typically include sugar alcohols (by reduction of a monosaccharide, an aldehyde group and a ketone group yield an alcohol group, and the monosaccharide yields a polyhydric alcohol having the same carbon number), uronic acids (oxidation of an alcohol group of a monosaccharide yields a uronic acid; D-glucuronic acid, galacturonic acid, and mannuronic acid are known in nature), and amino sugars

(substitution of an OH group with an NH₂ group of a saccharide molecule yields an amino sugar; glucosamine, chondrosamine, glycosides, and the like are known), but are not limited thereto. The derivative of D-allose is a D-allose derivative selected from a sugar alcohol in which a carbonyl group of D-allose is substituted with an alcohol group, a uronic acid in which an alcohol group of D-allose is oxidized, or an amino sugar in which an alcohol group of D-allose is substituted with an NH₂ group.

In the treatment composition of the present invention containing D-allose and/or a derivative thereof and/or a mixture thereof, the D-allose and/or the derivative thereof and/or the mixture thereof is contained in the composition in an effective amount. The "effective amount" is any amount sufficient for an intended purpose (for example, a desirable biological or medical response in a tissue or a subject).

The drug product of D-allose or a pharmacologically acceptable salt and/or hydrate of the present invention will be described. A liquid drug product exclusively containing D-allose and/or a derivative thereof and/or a mixture thereof may be used, or a liquid drug product further containing an appropriate additive such as a stabilizer and a preservative may be used. As the pharmaceutical composition of the present invention, a liquid containing an active component and a medically acceptable additive such as a carrier and a lubricant dissolved, for example, in water or various infusion preparations can be produced by a known formulation technique.

At least one of the antiproliferative agents is an anthracycline. The anthracycline is selected from the group consisting of doxorubicin, epirubicin, daunorubicin, aclarubicin, idarubicin, pirarubicin, annamycin, methoxymorpholinodoxorubicin, cyanomorpholinyldoxorubicin, valrubicin (N-trifluoroacetyladriamycin-14-valerate), and mitoxantrone. The anthracycline is selected from the group consisting of valrubicin, doxorubicin, and epirubicin. The anthracycline is epirubicin.

According to a preferred embodiment of the pharmaceutical product in a solution state of the present invention, the urothelial cancer therapeutic agent of the present invention is preferably dispersed in a solvent into a dispersion liquid (solution). Hence, the solution of the present invention can be used as a therapeutic agent that is efficiently administered into the renal pelvis and ureter or into the bladder by injection therapy into the urinary tract organ cavity of a patient. The dispersion liquid (solution) may have any pH, and high dispersibility can be achieved in a wide pH range of 3 to 10. From the viewpoint of administration safety in the body, the dispersion liquid preferably has a pH of 5 to 9, more preferably 5 to 8, and particularly preferably a neutral pH. According to a preferred embodiment of the present invention, the solvent is preferably an aqueous solvent and more preferably a pH buffer solution or a physiological saline. The aqueous solvent preferably has a salt concentration of 2 M or less and more preferably 200 mM or less from the viewpoint of administration safety in the body. The therapeutic agent in a solution state of the present invention is preferably contained at 10 mM or more relative to the dispersion. By continuous perfusion or retention for a certain period of time in the urinary tract organ cavity, the therapeutic agent is in contact with the renal pelvis and ureter or a large area of the bladder surface, and this enables treatment of multiple urothelial cancer. This treatment is also effective on urothelial cancer cells that cannot be removed by treatment such as surgery and should prevent recurrence. The solution not incorporated into cancer cells in the urinary tract organ cavity can be discharged outside the body together with urine.

As a water-soluble or water-swellable polymer, gelatin, a cellulose derivative, an acrylic acid derivative, povidone, macrogol, a polyamino acid derivative, or a polysaccharide is preferred. The gelatin is preferably purified gelatin; the cellulose derivative is preferably methyl cellulose, hydroxypropyl methyl cellulose 2910, hydroxypropyl methyl cellulose 2208, hydroxypropyl methyl cellulose 2906, hydroxypropyl cellulose, low-substituted hydroxypropyl cellulose, or carmellose sodium; the acrylic acid derivative is preferably an aminoacrylic methacrylate copolymer or a methacrylic acid copolymer; and the polyamino acid derivative is preferably polylysine or polyglutamic acid. The polysaccharide is particularly preferably hyaluronic acid, dextran, or dextrin. The amount of the water-soluble or water-swellable polymer varies with the characteristics and amount of D-allose, a derivative thereof, or a pharmacologically acceptable salt, the characteristics and molecular weight of the water-soluble or water-swellable polymer, or application sites, but the water-soluble or water-swellable polymer can be used in an amount of about 0.01% to 10% relative to the total amount of the drug product.

As a pH adjuster, an acid or an alkali harmless to the human body can be used, and as a surfactant, a nonionic surfactant, an anionic surfactant, or an amphoteric surfactant can be used. Examples of the osmotic pressure regulator include sodium chloride and glucose, examples of the antiseptic agent include parabens, and examples of the preservative include ascorbic acid and sulfites. The amounts of these additives are not specifically limited, and each additive can be used in such an amount as to achieve activities. As necessary, a local anesthetic such as procaine hydrochloride, a soothing agent such as benzyl alcohol, a chelating agent, a buffer, a water-soluble organic solvent, or the like can be added.

The "drug" or the "anticancer agent" of the present invention is administered to a cancer or precancerous tissue for treatment. Examples thereof include radioisotopes (such as iodine-131, lutetium-177, rhenium-188, and yttrium-90), toxins (such as diphtheria, Pseudomonas, ricin, and gelonin), enzymes, prodrug-activating enzymes, radiosensitizers, interfering RNAs, superantigens, antiangiogenic agents, alkylating agents, purine antagonists, pyrimidine antagonists, plant alkaloids, intercalating antibiotics, aromatase inhibitors, antimetabolites, mitotic inhibitors, growth factor inhibitors, cell cycle inhibitors, topoisomerase inhibitors, biological response modifying agents, antihormones, and antiandrogens.

The drug associated with D-allose (for example, binding or interaction) is used. The association may be covalent binding or noncovalent binding. D-Allose is associated with the drug at a strength sufficient to prevent disassociation before or during uptake into urothelial cancer cells, and any chemical, biochemical, or enzymatic coupling known to a person skilled in the art can be used.

When D-allose is associated with the drug through noncovalent binding, the association manner includes hydrophobic interaction, electrostatic interaction, dipole-dipole interaction, van der Waals interaction, and hydrogen bonding, and when D-allose is associated with the drug through covalent binding, D-allose and the drug is bonded directly or indirectly through a linker. Such covalent binding is achieved through an amide, ester, carbon-carbon, disulfide, carbamate, ether, thioether, urea, amine, or carbonate bond.

The safety of D-allose required for use as a pharmaceutical component is probably sufficient because the rare sugar is a monosaccharide present even in a trace amount in nature. Mutagenicity test, biodegradability test, and three acute toxicity tests (acute oral toxicity test, primary skin irritation test, and primary eye irritation test) are established as the most basic safety tests. The inventors ordered the basic safety tests of D-allose to a designated laboratory, and the result revealed sufficient safety.

The subject of the treatment composition of the present invention includes animals including humans (mammals such as humans, cattle, pigs, dogs, and cats; and birds such as fowls). The cancer cells as the target of the treatment composition of the present invention are urothelial cancer cells, and examples of the cell line include urothelial cancer cell lines (RT112, 253J, and J82).

### [Examples]

The present invention will next be described in further detail with reference to examples. The present invention is not intended to be limited thereto.

### (Examples)

### [Analysis of antitumor effect of rare sugar on human urothelial cancer cell lines]

Three human urothelial cancer cell lines (RT112, 253J, and J82) were used to analyze the antitumor effect of rare sugars. Ten rare sugars of L-allulose, D-allulose, D-allose, L-fructose, D-mannose, L-sorbose, D-tagatose, D-galactose, D-sorbose, and L-tagatose were used, and D-glucose and D-fructose were used as monosaccharides other than the rare sugars.

### [Used culture medium]

A minimum essential medium (MEM) containing D-glucose at 1,000 mg/l was used as the culture medium, and the antitumor effect of a rare sugar was evaluated in a prepared MEM containing a monosaccharide or a rare sugar at 10 mM, 25 mM, or 50 mM [in the drawings, indicated by (10), (25), or (50)]. An MEM containing no sugar was used for control.

### [Experimental procedure]

With the MEM, cell suspensions each at 5.0 × 10⁴ cells/ml were prepared. Each suspension was distributed in a 96-well plate at 0.1 ml/well and then was incubated for 24 hours. The culture solution was then removed, next a culture solution containing a monosaccharide or a rare sugar at 10 mM, 25 mM, or 50 mM in the MEM was added at 0.1 ml/well, and the whole was incubated for 24 hours (#1 to #13).
#1: Only the MEM solution (in the drawings, Control)
#2: A D-glucose solution (in the drawings, D-Glucose)
#3: An L-allulose solution (in the drawings, L-Allulose)
#4: A D-allulose solution (in the drawings, D-Allulose)
#5: A D-fructose solution (in the drawings, D-Fructose)
#6: A D-allose solution (in the drawings, D-Allose)
#7: An L-fructose solution (in the drawings, L-Fructose)
#8: A D-mannose solution (in the drawings, D-Mannose)
#9: An L-sorbose solution (in the drawings, L-Sorbose)
#10: A D-tagatose solution (in the drawings, D-Tagatose)
#11: A D-galactose solution (in the drawings, D-Galactose)
#12: A D-sorbose solution (in the drawings, D-Sorbose)
#13: An L-tagatose solution (in the drawings, L-Tagatose)

### [Cell survival assay (MTT assay)]

An MTT assay is a test method for determining survival and uses a color reaction of an insoluble formazan pigment (blue) formed by reduction of MTT as a tetrazolium salt. The MTT is reduced by succinate-tetrazolium reductase as a reductase of mitochondria. Living cells have high enzyme activity to exhibit coloration, but the coloration is not observed when cell death including apoptosis occurs. The coloration is measured with a microplate reader to determine the cell survival rate.

### [Result of cell survival assay]

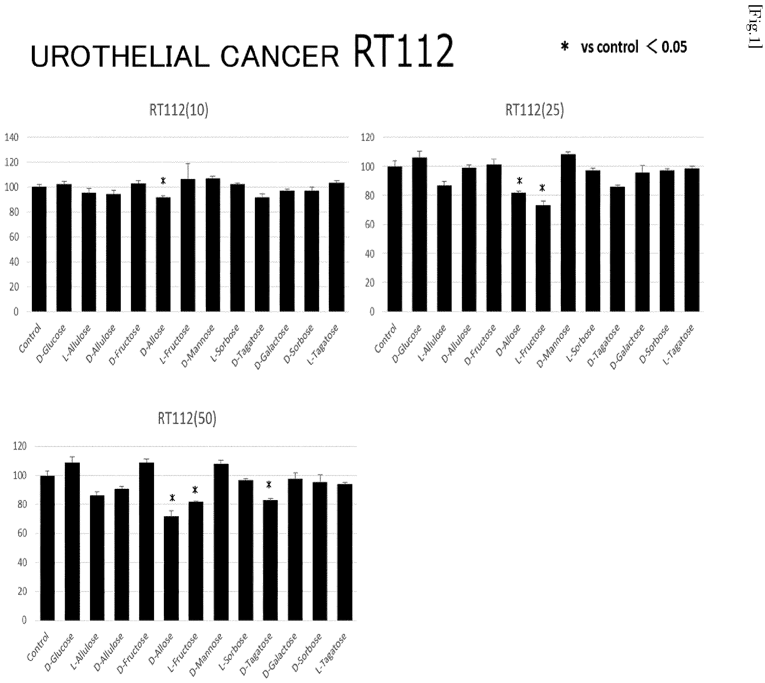

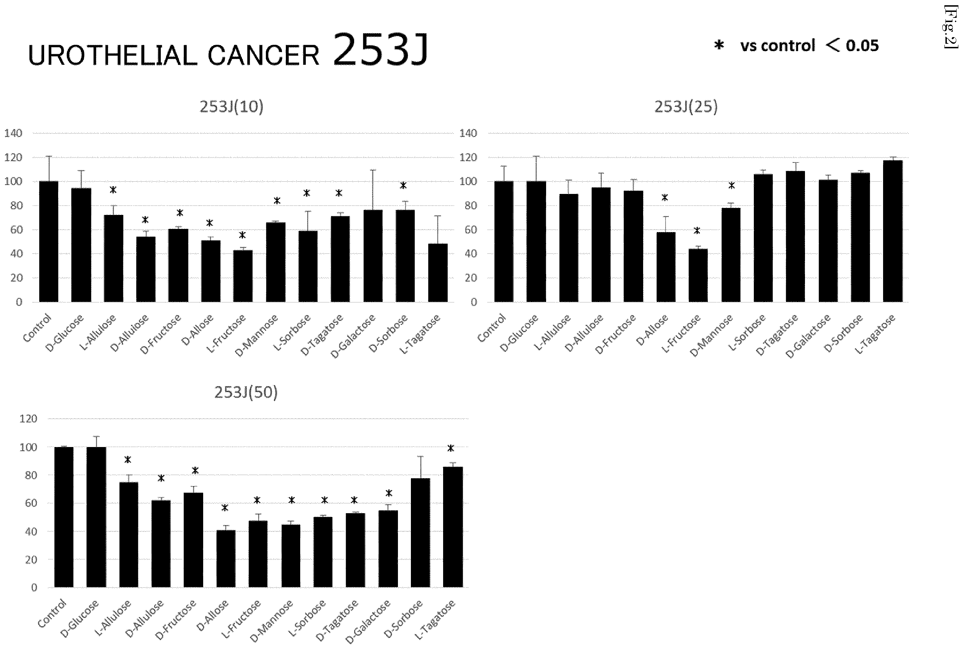

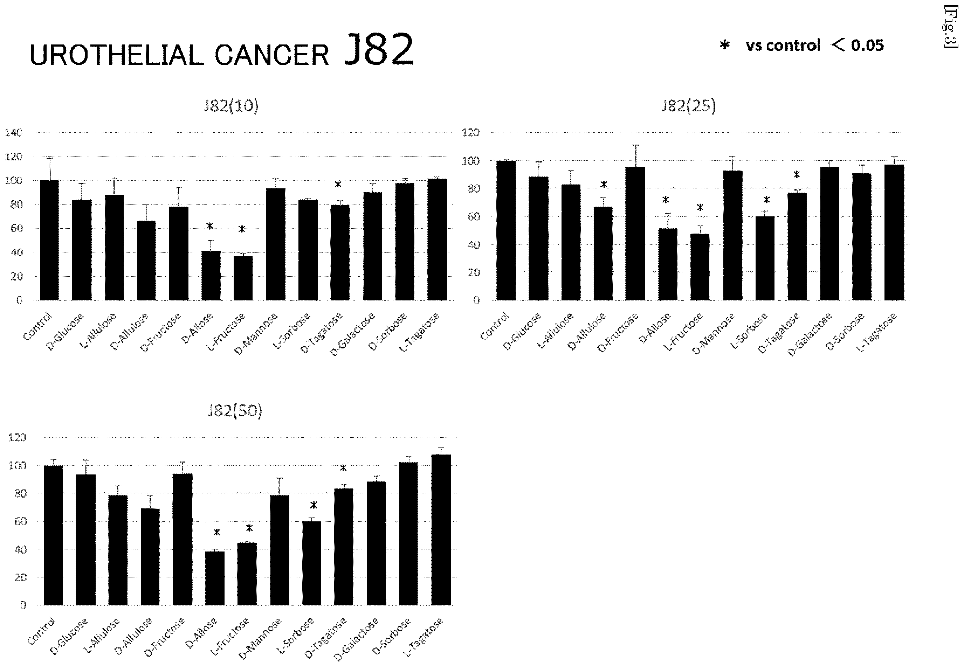

Changes in survival rate of human urothelial cancer cells co-cultured with a rare sugar are shown in Fig. 1 to Fig. 3. In other words, Fig. 1 shows the result of survival assay of a urothelial cancer cell line (RT112), Fig. 2 shows the result of survival assay of a urothelial cancer cell line (253J), and Fig. 3 shows the result of survival assay of a urothelial cancer cell line (J82). As shown in Fig. 1 to Fig. 3, in all the cell lines, D-allose significantly reduced the survival rate at each concentration of 10 mM, 25 mM, and 50 mM as compared with the control (p < 0.05) and exerted the strongest antitumor effect among the analyzed sugars.

### [Discussion]

In cancer cells, ATP production in mitochondria is suppressed. In cancer cells, a metabolic system called "glycolysis" in which ATP is produced from glucose without oxygen is enhanced. The glycolysis proceeds in cytoplasm. Cancer cells suppress aerobic respiration in mitochondria for some reasons.

One reason is that a large amount of glucose is required as a material for synthesizing cell components. For cell proliferation by cell division, cell components such as nucleic acids, cell membranes, and proteins are required to be newly produced. Cells can produce nucleic acids, lipids, and amino acids from glucose through glycolytic pathway and various intracellular metabolic pathways stemming therefrom. If all glucose were used with oxygen to produce ATP in mitochondria, materials for cell production would be consumed.

Aerobic respiration in mitochondria accelerates the production of active oxygen. The active oxygen damages cells, inhibits proliferation or metastasis, and can induce cell death. Cancer cells seem to suppress the use of oxygen in mitochondria so as not to accelerate the production of active oxygen. To cancer cells, suppression of the metabolism using oxygen in mitochondria is advantageous for survival or proliferation.

In the case of cancer cells, it is known that when cancer cells have higher mitochondrial activity, proliferation or metastasis is suppressed, and cell death is induced. This is because complete decomposition of glucose results in insufficient materials for cell proliferation, an enhancement of aerobic respiration accelerates the production of active oxygen, and damage by the active oxygen induces cancer cell suicide. In other words, a treatment method of activating mitochondria of cells can kill only cancer cells while enhancing normal cell function. D-Allose reduces the survival rate of all the human urothelial cancer cells at 10 mM or higher and seems to exert antitumor effect.

### Industrial Applicability

The research result of the present invention has revealed antitumor effect of D-allose on urothelial cancer cells by in-vitro experiments using a plurality of human urothelial cancer cell lines for the first time and has suggested that D-allose is probably to be a key drug for treatment of urothelial cancer in the future. Meanwhile, the injection therapy into the urinary tract organ cavity has such an organ specific advantage that a medicinal agent can be directly applied to cancer cells, and the injection therapy using existing medicinal agents has still failed to achieve satisfactory treatment outcomes. In addition, patients with non-muscle invasive urothelial cancer as the treatment subject are extremely numerous, and thus the ureteropelvic injection therapy or the intravesical injection therapy using D-allose is a novel treatment method proposed on the basis of such a revolutionary idea that D-allose having the antitumor effect is directly applied to urothelial cancer cells, and is a novel treatment method that should probably make a large impact on the society and should probably be applied in clinical practice in the near future.

## Claims

1. A solution for injection into a urinary tract organ cavity, the solution comprising: D-allose.

2. The solution according to claim 1, wherein the D-allose is D-allose and/or a derivative thereof and/or a mixture thereof.

3. The solution according to claim 2, wherein the derivative of D-allose is a D-allose derivative selected from a sugar alcohol in which a carbonyl group of D-allose is substituted with an alcohol group, a uronic acid in which an alcohol group of D-allose is oxidized, or an amino sugar in which an alcohol group of D-allose is substituted with an NH₂ group.

4. The solution according to any one of claims 1 to 3, wherein an inside of the urinary tract organ cavity is an inside of an upper urinary tract and an inside of a bladder.

5. The solution according to any one of claims 1 to 4, for injection therapy into the urinary tract organ cavity.

6. The solution according to any one of claims 1 to 5, using enhancement of sugar uptake into cancer cells of the urinary tract organ by D-allose.

7. The solution according to any one of claims 1 to 6, wherein D-allose is contained in an effective amount.

8. The solution according to any one of claims 5 to 7, to be injected into the urinary tract organ cavity by injection into the urinary tract organ cavity.

9. The solution according to any one of claims 1 to 8, wherein D-allose is contained together with a pharmaceutically acceptable diluent or a pharmaceutically acceptable carrier.

10. The solution according to any one of claims 1 to 9, wherein one or more antiproliferative agents are further contained.

11. The solution according to any one of claims 1 to 10, wherein D-allose is associated with a drug for enhancing uptake into cancer cells of the urinary tract organ.

12. The solution according to claim 11, wherein the drug includes an anticancer agent.

13. The solution according to claim 11 or 12, wherein D-allose and the drug are associated directly or covalently through a linker.

14. The solution according to any one of claims 11 to 13, wherein the drug is a radioisotope, an enzyme, a prodrug-activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

15. The solution according to claim 10, wherein at least one of the antiproliferative agents is an anthracycline.

16. The solution according to claim 15, wherein the anthracycline is selected from the group consisting of doxorubicin, epirubicin, daunorubicin, aclarubicin, idarubicin, pirarubicin, annamycin, methoxymorpholinodoxorubicin, cyanomorpholinyldoxorubicin, valrubicin (N-trifluoroacetyladriamycin-14-valerate), and mitoxantrone.

17. The solution according to claim 15, wherein the anthracycline is selected from the group consisting of valrubicin, doxorubicin, and epirubicin.

18. The solution according to claim 15, wherein the anthracycline is epirubicin.

19. The solution according to any one of claims 6 to 18, wherein the urinary tract organ cancer is non-muscle invasive urothelial cancer.

20. The solution according to any one of claims 6 to 19, wherein the urinary tract organ cancer is renal pelvic cancer, ureter cancer, bladder cancer, or ureteral cancer.

21. The solution according to any one of claims 1 to 20, being a pharmaceutical composition for prevention or treatment of urinary tract organ cancer.

22. A kit comprising:
the solution as a pharmaceutical composition according to claim 21; and
a package insert indicating that the pharmaceutical composition is injected into a urinary tract organ cavity of a patient requiring prevention or treatment of urinary tract organ cancer, and accordingly the urinary tract organ cancer of the patient is prevented or treated.
